(19) Europäisches Patentamt
European Patent Office
Office européen des brevets

(11) Publication number: **0 447 836 A2**

# (12) EUROPEAN PATENT APPLICATION

(21) Application number: **91102686.2**

(22) Date of filing: **23.02.91**

(51) Int. Cl.[5]: **C12N 15/12**, C07K 15/00, C12Q 1/68, A61K 37/02

(30) Priority: **12.03.90 US 492517**
**03.08.90 US 562177**

(43) Date of publication of application:
**25.09.91 Bulletin 91/39**

(84) Designated Contracting States:
**AT BE CH DE DK ES FR GB GR IT LI LU NL SE**

(71) Applicant: **AMERICAN CYANAMID COMPANY**
**1937 West Main Street P.O. Box 60**
**Stamford Connecticut 06904-0060(US)**

(72) Inventor: **Vitek, Michael Peter**
**213 Wyckoff Avenue**
**Waldwich, New Jersey 07463(US)**
Inventor: **Jacobsen, Jack Steven**
**111 East Clifton Avenue**
**Clifton, New Jersey 07011(US)**

(74) Representative: **Wächtershäuser, Günter, Dr.**
**Tal 29**
**W-8000 München 2(DE)**

## (54) Amyloid peptide precursor RNA.

(57) A human amyloid peptide precursor RNA from the brains of persons afflicted with Alzheimer's disease and Down's syndrome is described. The RNA is virtually absent from mouse or rat brain tissue yet is abundant in human brain tissue. A cDNA clone corresponding to the RNA predicts the existence of a 365 amino acid protein that is similar to the amino-terminal end of an amyloid peptide precursor previously described (APP 770), but lacks the beta-amyloid peptide or any hydrophobic transmembrane spanning domains. These features suggest that the human RNA codes for a soluble protein containing a Kunitz protease inhibitor.

EP 0 447 836 A2

BACKGROUND OF THE INVENTION

The present invention relates to a novel human amyloid peptide precursor RNA isolated from the brains of persons afflicted with Alzheimer's disease and Down's syndrome.

Alzheimer's disease is a degenerative disorder of the human central nervous system characterized by progressive impairment of memory and intellectual function, usually beginning in middle to late adult life. The disease affects about 10% of the population aged 65 years and older (Hardy, J.A., et all, 7 Neurobio Aging 489 (1986)), and is the leading cause of dementia and among the leading causes of death in the United States (Katzman, R.,33 Arch. Neurol.217 (1976)).

The cause of Alzheimer's disease is unknown. The disease exists worldwide, and nothing suggests a clear relationship to nutritional factors, infection, toxins, or other environmental factor. A history of affected family members occurs in about a quarter of the cases. This, plus the fact that almost all patients with Down's syndrome develop Alzheimer's changes in the brain, suggest a genetic susceptibility, perhaps transmitted as an autosomal dominant trait on chromosome 21 (St. George-Hyslop, P.H., et al., 235 Science 885 (1987); see also the summary in Wyngaarden, J.B., and Smith, L.H., eds., Cecil's Textbook of Medicine, 18th ed., W.B. Saunders, 1988, pages 2089-90). Yet other influences must be important, for identical twins show less than 50% concordance for illness, and, when such cases have appeared, several years often separate the onset of signs and symptoms in the affected pair (ibid.).

Alzheimer's disease produces a progressive neuronal degeneration of selective cells in the association and memory areas of the cerebral cortex, combined with similar abnormalities in certain subcortical nuclei. Neuronal loss especially affects the large pyramidal cells of the hippocampus, the amygdala and the parietal and frontal association area. Histopathologically, many of the degenerating nerve cells contain tangles of twisted intracellular fibrils of a unique protein configuration related to altered neurofibrillary protein (Cecil's, supra), and amyloid plaques and cerebrovascular amyloid deposits comprising extracellular insoluble fibrillar proteins occur in abnormally large numbers (Hardy, et al., supra, and Wong, C., et al., 82 proc. Nat. Acad. Sci. U.s.A. 8729 (1982)). These findings have become a hallmark of the disease.

The pathologic changes of Alzheimer's disease appear to be more quantitatively than qualitatively different from those observed in other disorders, including uncomplicated aging. Neuronal loss, plaques, and tangles, usually in lesser degrees, can be found in the brains of many intellectually intact elderly persons. However, the degree of intellectual impairment in patients appears to be correlated with the frequency of neuritic plaques in the cortex (Roch, M., et al., 209 Nature 109 (1966)).

Alzheimer's disease usually begins insidiously, and hints of abnormal behavior may appear several years before sustained clinical changes allow a diagnosis. Diagnoses are generally made differentially, by exclusion. Psychologic depression, drug intoxication, some of the diffuse angiopathies, metabolic deficiencies, chronic meningitis, Pick's disease, or early stages of diffuse primary or metastatic brain tumors may create diagnostics errors. No specific laboratory determinants for Alzheimer's disease exist (Cecil's, supra at page 2090).

There is no specific treatment for Alzheimer's disease, although inosine dialkylaminoalcolo derivatives have been suggested to counter somewhat the effects on memory and learning loss (U.S. Pat. No. 3,646,007 to Gordon), and administration of certain drugs that selectively promote or enhance neurotransmission have been suggested to reduce symptoms (U.S. Pat. No. 4,469,707 to Lindberg and Oegren, U.S. Pat. No. 4,511,570 to Tuttle, and U.S. Pat. No. 4,711,891 to Aufdembrinke, et al.). But neither diet nor drugs have been shown to slow the steady intellectual deterioration. The brunt of care usually falls on family or social agencies during the early and intermediate stages of illness. Institutionalization often is required in late stages, when patients may sink to a purely vegetative level.

The tragedy of Alzheimer's disease is exacerbated by the likelihood that it will occur more frequently as the relative size of elderly populations in societies throughout the world increases. Hence, major research efforts seek treatments and cures, as well as diagnostic and management aids.

Recent studies have begun to elucidate the biochemical basis of the disorder. A 42-amino acid peptide has been isolated as the major constituent of brain amyloid plaques and deposits of patients afflicted with Alzheimer's disease (Glenner, G.G., and Wong, C., 112 Biochem. Biophys. Res. Commun. 1131 (1984) and Masters, C., et al., 82 Proc. Nat. Acad. Sci. U.S.A. 4245 (1985)). This peptide has been sequenced, and has a partial beta-pleated sheet structure; hence, it is called a beta-amyloid peptide or BAP. Isolates of beta-amyloid peptides from the brains of Down's syndrome patients are similar, having an identical 28-amino acid stretch, indicating a common mechanism underlying amyloidosis in both disorders (Tanzi, R., et al., 331 Nature 528 (1988)).

Molecular genetic studies (primarily cDNA cloning, but also immuno-staining) indicate that beta amyloid peptide is derived from a larger, amyloid precursor protein, called APP. Using a fetal brain library and an

oligonuoleotide probe drawn from the amino acid sequence, what appears to be a full length cDNA clone with 695 amino acid reading frame for the putative precursor protein has been isolated and called APP 695 (Kang, J., et al, 325 Nature 733 (1987)). Other amyloid peptide precursor cDNAs with open reading frames have been subsequently isolated from other libraries: a 751 amino acid putative precursor (APP 751) both from an SV 40 transformed fibroblast library and a promyelocytic leukemia cell line (HL60) library (Ponte, P., et al. 331 Nature 525 (1988) and Tanzi, supra, respectively), and a 770 amino acid putative precursor (APP 770) from a human glioblastoma library (Kitaguchi, N., et al., 331 Nature 530 (1988)). More recently, a fourth protein precursor, APP 563, was described (DeSauvage, F., and Octave, J.N., 245 Science 651 (1989); it has the 208 amino acid carboxy terminal containing the BAP domain of APP 751 replaced with a novel 20 amino acid insert. These similar but nonidentical precursors probably arise through differential splicing of transcripts (Tanzi and Ponte, supra, and Lemaire, H.G., et al, 17 nucleic Acid Res. 517 (1989)).

It has been suggested that overproduction of amyloid peptide in Alzheimer's patients may occur because of a frameshift mutation in the gene encoding for the precursor protein, causing repeated translation of the peptide (DEr. Pat. Ap. No. 341,491 to Scangos, et al.). This mutation is contemplated to occur between nucleotides 1897 and 1921 of the cDNA sequence published by Kange, et al, supra.

The open reading frames of amyloid precursor protein DNA appear to code for other functional elements in addition to beta-amyloid peptides. Both APP 751 and APP 770 contain 56 amino acid domains which share structural and functional similarity with the Kunitz domain of serine protease inhibitors. Kunitz inhibitors are a family of polypeptide inhibitors specific for serine proteases such as trypsin (reviewed by Laskowski, M., and Kato, I., 49 Ann. Rev. Biochem. 593, 610-613 (1980)). The physiological function of Kunitz-type inhibitors is not understood, but it has been suggested that the presence of the domain alters the metabolism of the amyloid precursor protein, perhaps protecting the molecule from degration by serine proteases (Tanzi, r.E., et al, supra). Computer modelling and in vitro analyses strongly suggest that APP domains also contain structural transmembrane spanning anchor domains (Selkoe, D.J., et al., 85 Proc. Nat. Acad. Sci. U.S.A. 7341 (1988) and Dyrks, T., et al., 7 EMBO 949 (1988)).

An understanding of the biochemical events leading to the deposition of amyloid peptides in brain tissue is complicated by other findings. Amyloid precursor protein RNAs and proteins appear to be present in rodents (shown by Northern blots, cDNA cloning and innumo-staining), even though amyloid plaques in their brains have not been found (Kang, J., and Muller-Hill, B., 17 Nucleic Acid Res. 2130 (1989), Bendotti, C., et al., 85 Proc. Nat. Acad. Sci. U.S.A. 3628 (1988), and Card, J.P., et al., 1 neuron 835 (1988)). Also, additional APP transcripts which are not accounted for by described cDNA clones appear to exist, including multiple forms of amyloid precursor protein present within a single cell type (shown by hybridization of APP cDNA clones to Northern blots of Hl60 cells and human brain RNAs; see Donnelly, R.J., et al., 9 Neurobiol. Aging 333 (1988), Vitek, M.P., et al., 4 Mol. Brain Res. 121 (1988), and Zain, S.B., et all, 85 Proc. Nat. Acad. Sci. U.S.A. 929 (1988)).

## SUMMARY OF THE INVENTION

This invention is based upon the finding of a species specific, novel amyloid peptide precursor RNA from human brain tissue of Alzheimer's disease patients. Except for an exact match between positions 115 and 805 and 818 and 1090 with that described for APP 770 (numbering according to Kitaguchi, et al., and Donnelly, et. al. supra), the nucleotide sequence of complementary DNA cloned from the novel RNA of this invention lacks substantial homology to any known sequence, including the unique regions of the recently described APP 563 clone (DeSauvage and Octave, supra).

Quantitatively, the novel RNA of this invention represents a major APP RNA in human brain, four-fold more abundant than APP 770, at least 31-fold more abundant than APP 751, less abundant than APP 695, and virtually absent from mouse and rat brain RNAs.

A 1.6 kilo-basepair cDNA clone corresponding to the novel APP RNA predicts the existence of a 365 amino acid protein that is similar to the amino-terminal end of APP 770, but lacks the beta-amyloid peptide domain, or any hydrophobic transmembrane spanning domains. These features suggest that the novel APP RNA codes for a soluble protein contacting a Kunitz protease inhibitor.

The present invention thus provides not only a novel human amyloid precursor RNA, but also complementary DNA made using the novel RNA as a template, and means to obtain novel purified and isolated amyloid protein precursor products by transforming or transfecting cells with the novel RNA or cDNA of this invention.

## BRIEF DESCRIPTION OF THE DRAWINGS

Figure 1 schematically illustrates (A) and SI nuclease protection analysis employed to probe APPs; (B) predicted protection schemes using the analysis with known APPs; and (C) a deduced protection scheme for the novel RNA of this invention. Figure 2 shows autoradiographs of SI nuclease protection analysis (top) and comparisons of mobilities of APP cDNA clones subjected to SI nuclease protection analysis (bottom). Figure 3 schematically shows (A) the organization of the nucleotide and predicted amino acid sequence of a novel APP cDNA of this invention; (B) the first 670 nucleotides of the novel insert; and (C) the complete nucleotide and predicted amino acid sequence of a cDNA corresponding to a novel RNA of this invention.

## DETAILED DESCRIPTION OF THE INVENTION

This invention relates to a major, species-specific human amyloid peptide precursor RNA isolated from brain tissue of patients afflicted with Alzheimer's disease. More particularly, this invention describes a novel amyloid peptide precursor RNA coding for a protein comprising fewer than 500 amino acids. The sequence of a 1.6 kilo-basepair cDNA clone corresponding to the novel RNA of this invention predicts a soluble protein comprising a Kunitz protease inhibitor and lacking both beta-amyloid peptide and a hydrophobic transmembrane spanning anchor.

The novel RNA of this invention has been observed in all preparations of human RNAs from multiple individuals examined, and accounts for 18 to 24% of all amyloid precursor protein (APP) RNAs. Quantitatively, it is less abundant than previously described APP 695, but more abundant than APP 770. The novel RNA of this invention appears to be unique to human beings; rat and mouse brain RNAs have no detectable levels. Moreover, both previously described APP 563 and APP 751, present in rodents, appear to be absent or at extremely low levels in the cortex of adult human brains.

Complementary DNA to the novel RNA of this invention predicts that the RNA of this invention codes for a protein comprising about 365 amino acids. Therefore, as used herein, the novel RNA of this invention shall be referred to as coding for APP 365.

The nucleotide sequence of complementary DNA to the novel RNA of this invention lacks substantial homology to any known sequence in various databanks (such as GenBank (release 59.0, 3/89) and GenEMBL (release 18.0, 2/89). Databanks, were searched using the sequence analysis software package by the "Genetics Computer Group" of the University of Wisconsin according to protocols described in Devereaux, J., et al. 12 Nucleic Acids Res. 387 (1986)), including the unique regions of the recently described APP 563 cDNA clone, except for an exact match between positions 115 and 805 of previously described APP 770, and also between 818 and 1090 (numbering according to Kitaguchi, et al, and Donnelly, et al., supra). Complementary DNA to the novel RNA of this invention contains a new "insert" sequence starting after position 1090 of the APP 770 nucleotide sequence, and lacks the 12 basepairs found between positions 805 and 818.

This is schematically illustrated in Figure 3B, which depicts the organization and nucleotide sequence of the novel RNA of this invention in comparison with previously described APP 770. The figure is oriented in a conventional 5' to 3' direction. Nucleotide positions 1 to 865 are according to the numbering system of Kang (Kang, J., et al. supra). The Kunitz protease inhibitor (PI, positions 866-1033, and C, positions 1034-1090) domains are as described by Kitaguchi, et al. and Donnelly, et al., supra. The 12-nucleotide deletion (positions 806-817) and a new carboxy-terminus (positions 1091-1760) are indicated.

A complete nucleotide sequence of a complementary DNA to the RNA of this invention beginning at position 115 according to Kang (supra) numbering system and continuing for 1630 additional nucleotides is given in Figure 3C. The 12-nucleotide deletion is shown with a vertical arrow between nucleotides 805 and 806. Double underlined residues represent the Kunitz protease inhibitor region, and single underlined residues represent the novel carboxy-terminus.

The amino acid sequence deduced from the cDNA suggests the novel RNA of this invention codes for a soluble, shortened form of a Kunitz protease inhibitor-contacting APP protein. The start of the variant amino acid sequence corresponds to residue $Gly^{39}$ and continues to exactly match APP 770 until residues $Thr^{270}$-$Ser^{271}$-$Ile^{272}$-$Ala^{273}$, which are absent. Following this four-residue deletion, the novel RNA continues with the exact Kunitz protease inhibitor domain and the 19-amino acid carboxy-terminal to the Kunitz protease inhibitor domain as found in APP 770, but then terminates with six unique amino acid residues.

Whereas the Kunitz protease inhibitor domain is present, the novel RNA of this invention lacks the long hydrophobic transmembrane-spanning anchor or the beta-amyloid domain found in other amyloid precursor proteins. The APP 365 of this invention may be related to immunoprecipitable 35 kiloDalton APP protein found to be four-fold more abundant in Alzheimer's disease brains than in normal brains (Cole, G., et al., 100 Neurosci. Lett. 340 (1989)).

The novel RNA of this invention provides a soluble, diffusible protein which, whether secreted or released by proteolysis from membrane bound APP protein, plays a role in the amyloidosis of Alzheimer's disease. Thus, this invention provides soluble polypeptides wholly or partially duplicative of continuous sequences of species-specific amyloid peptide precursor amino acid residues herein elucidated for the first time. These sequences, by virtue of sharing primary, secondary, or teriary structural and conformational characteristics with naturally-occurring amyloid peptide precursor proteins, may possess biological activity and/or immunological properties in common with the naturally-occurring product such that they, and/or their antibodies, may be employed as biologically active or immunological substitutes for amyloid precursor protein in diagnostic, thereapeutic, and immunological processes.

As described above, illustrating the present invention are cloned complementary DNA sequences defining exons of human polypeptide sequences suitably deduced therfrom, which represent the primary structural conformation of a major amyloid precursor protein of human origin. Also encompassed are DNA sequences homologous or closely related to complementary DNA described herein, namely DNA sequences which hybridize, particularly under stringent conditions, to DNA complementary to the RNA of this invention, and DNA sequences encoding proteins sufficiently duplicative of the protein encoded by DNA complementary to the RNA of this invention to allow possession of the biological properties of said protein. Thus, this invention includes, but is not limited to, RNA and DNA sequences encoding a soluble Kunitz protease inhibitor domain of human brain amyloid precursor proteins.

Having herein elucidated the sequence of amino acid residues of a major amyloid precursor protein of human origin and cloned complementary DNA corresponding thereto, this invention also provides novel, biologically functional viral and circular plasmid RNA and DNA vectors incorporating RNA and DNA sequences of the invention generated by standard means. Microbial (e.g., bacterial, yeast and mammalian cell) host organisms may be stably transformed or transfected with such vectors. Correspondingly provided by the invention are novel methods for the production of useful polypeptides comprising cultured growth of such transformed or transfected microbial hosts under conditions facilitative of large scale expression of the exogenous, vector-borne DNA or RNA sequences, and the isolation of the desired polypeptides from the growth medium, cellular lysates, or cellular membrane fractions. An example of such expression is set out as Example 4.

Isolation and purification of microbially expressed polypeptides provided by the invetion may be by conventional means including, for example, preparative chromatographic separations and immunological separations, including monoclonal and/or polyclonal antibody preparations.

The present invention provides for the total and/or partial manufacture of DNA sequences coding for amyloid precursor protein 365 and homologous proteins, and including such advantageous characteristics as incorporation of codons preferred for expression by selected non-mammalian hosts, provision of sites of cleavage by restriction by endonuclease enzymes, and provision of additional initial, terminal or intermediate DNA sequences which facilitate construction of readily expressed vectors. Correspondingly, the present invention provides for manufacture (and development by site specific mutagenesis of cDNA and genomic DNA) of DNA sequences coding for microbial expression of polypeptide analogues or derivatives of amyloid protein precursor which differ from naturally-occurring forms in terms of identity or location of one or more amino acid residues (i.e., deletion analogues containing less than all of the residues specified for APP 365, and/or substitution analogues wherein one or more residues are added to a terminal or medial portion of the polypeptide), and which share some or all of the properties of naturally-occurring forms.

Novel RNA sequences of this invention code for all sequences useful in securing expression in procaryotic or eucaryotic host cells of polypeptide products having at least a part of the primary structural conformation, and one or more of the biological properties of amyloid precursor protein 365, which are comprehended by: (a) the DNA sequences as set our in Figure 3C or complementary strands; (b) DNA sequences which hybridize (under hybridization conditions as described herein or more stringent conditions) to DNA sequences defined in (a) or fragments thereof; and (c) DNA sequences which, but for the degeneracy of the genetic code, would hybridize to the DNA sequences defined in (a) and (b) above. Specifically comprehended are genomic DNA sequences encoding allelic variant forms of amyloid precursor protein sequences encoding amyloid precursor protein RNA, fragments thereof, and analogues wherein RNA or DNA sequences may incorporate codons facilitating transcription or RNA replication of messenger RNA in non-vertebrate hosts.

Polypeptide products of the invention may be "labelled" by covalent association with a detectable marker substance (e.g., radiolabeled with $^{125}I$ or fluorescent antibody) to provide reagents useful in the detection and quantification of human beta-amyloid protein presursor protein in solid or liquid fluid samples such as tissue slices or spinal fluid. RNA or DNA products of the invention may also be labeled with detectable markers and employed in DNA hybridization processes to locate the beta-amyloid protein position and/or position of any related gene family in a mammalian species chromasomal map. They can also be used for identifying neighboring genes and their disorders.

Thus, this invention may be used for diagnosis (histopathology as well as clinical chemistry) and for research, and for biochemically-based treatments and cures. In particular, a role has been proposed for the extra-cellular action of the KPI domain of related proteins with respect to stimulation of neurite outgrowth (Monard, 1988, Zurn, 1988). Thus, the protein encoded by the RNA and DNA sequences of the present invention may also be useful in directly or indirectly promoting nerve growth in vivo and in vitro. It is therefore contemplated that the APP-365 protein or active portion thereof will be used in nerve cell maintenance, growth and repair, both as a supplement in nerve cell culture and as an active component of a therapeutic composition for in vivo treatment and repair of neural tissue damage.

The following examples are presented to further describe and explain the present invention and the characterization techniques employed, and should not be taken as limiting in any regard. Unless otherwise indicated, all parts and percentages are by weight, and are based on the weight at the particular stage of the processing being described.

## Example 1

### SI Nuclease Protection Strategy

SI nuclease from Aspergillus oryzae is a highly specific single-stranded endonuclease. SI nuclease applications (described more fully in Ausbel, F.M., et al. eds., Current Protocols in Molecular Biology, John Wily & Sons, New York, 1989, 3.12.1 to 3.12.3) make use of the ability of the enzyme to trim protruding single-stranded ends of DNA and RNA without significant nibbling of blunt duplex ends. Hybridized transcripts of complementary RNA and DNA, for example, are SI-resistant, and may be isolated and analyzed after SI digestion of adjacent noncomplementary domains.

This example employs this latter technique to simultaneously characterize the quality and quantity of several APP transcripts. A uniformly labeled anti-sense riboprobe derived from the 366 basepair Dde I-Xho I (positions 774 to 1139) fragment of APP 770 complementary DNA, described in Kitaguchi, et al., and Donnelly, et al., cited above, is prepared using the "Ribogenesis II, In Vitro Transcription Biosystem" kit (International Biotechnologies, Inc., New Haven, CT) using standard techniques (Sambrook, J., E.F., Fritsch, and T. Maniatis, Molecular Cloning: A Laboratory Manual, 2nd ed., Cold Spring Harbor Laboratory Press, 1989, 10.29 to 10.37). This probe permists the precise measurement of internal fragments of APP RNAs which include or flank the nucleotides of exon encoding the Kunitz protease inhibitor domain (Tanzi, et all, Kitaguchi, et al., and Ponte, et al., cited above).

The probe, which has 410 bases and is labelled with $^{32}P$, is depicted schematically in Figure 1A. pAPP770-DX represents a portion of a plasmid constructed by insertion of the 366-base Dde I-XhoI fragment (derived from an APP 770 cDNA clone) into the Sma I-XhoI sites of riboprobe vector pIBI30 (International Biotechnologies, Inc., New Haven, CT). Transcription of Eco R-linerized template with $T_3$ RNA polymerase generates a 410-base anti-sense riboprobe which includes sequences complementary to the Kunitz protease inhibitor (filled box), C (vertical hatched box) and flanking (open box) domains of APP-related RNAs, as well as additional vector sequences (stippled boxes). The riboprobe contains a total of 370 bases complementary to the APP770 transcript; length of releveant sequences are given.

The labeled riboprobe is then hybridized to human, rat, or mouse brain poly-(A+) (purchased from Clontech Laboratories, Inc., Palo Alto, CA) or HL60 ply-(A+) (prepared according to Furuya, H., et al. 150 Biochem. Biophys. Res. Commun. 75 (1988), Murdoch, G., et al. 16 Nucleic Acids Res. 357 (1988), Podlisny, M., et al. 238 Science 669 (1987), and St. GeorgeHyslop, P., et al., supra). The riboprobe is also hybridized to total cellular brain tissue or HL60 RNAs (prepared by the method of Chirgwin (Chirgwin, et al., 24 Biochem. 5294 (1979), which involves homogeniztion of tissue in guanidine isothiocyanate, overlay of the homogenate atop a cesium chloride cushion, and recovery of RNA by ultracentrifugation).

RNA (10-15 ug), excess radiolabelled riboprobe (3-5 x $10^5$ cpm) and E. coli t-RNA carrier (100 ug) are precipitated and resuspended in uL of hybridization buffer (80% formamide, 40mM PIPES (pH 6.4), 0.4 M NaCl, 1 mM EDTA), heated at 80°C for 5 min and annealed att 44°C for 16 hours. RNA is digested for 1 hr at 37°C with the addition of SI nuclease (300 units; Boehringer-Mannheim) in 0.3 ml buffer containing 280 mM NaCl, 50 mM NaOAc (pH 4.5), 4.5mM $ZnSO_4$, and heat denatured salmon sperm DNA (40 ug/ml).

This generates protected fragments illustrated in Figure 1B. APP RNAs hybridized to radiolabelled riboprobe are expected to generate SI-resistant probe fragments whose lenths of 370, 261, and 93 bases indicate the presence of APP 770, 751, and 695 RNAs, respectively.

Digestion is terminate with the addition of 80 ul stop buffer (4 M ammonium acetate, 20 mM EDTA (pH 8.0), and 40 ug/ml t-RNA). The samples are precipitated with ethanol, and the pellets are resuspended in 8 ul loading buffer (95% formamide, 20 mM EDTA, 0.05% bromphenol blue and 0.05% xylene cyanol FF). The samples are fractionated by electrophoresis on high resolution 6% polyacrylamide-8 M urea sequencing gels. SI-protected bands are visualized by autoradiography (Kodak XAR film).

Autoradiographs are shown in Figure 2 (top). Samples include total RNA from human brain temporal cortex (lanes 2 and 3); human promyelocytic leukemia (HL60) cells poly-(A+) RNA (lane 4); and human, mouse and rat brain poly-(A+) RNAs in lanes 5 to 5, respectively. Bands corresponding to APP 770, APP 695 and novel APP 365 are shown in lanes 2 to 3. In lanes 4 to 7, the APP 751 RNAs. Markers (lane 1) are $^{32}$P-labelled HaeIII restriction fragments from phiXl74. (In this system, the protected RNA fragments derived from the labeled riboprobe migrate faster than the labeled DNA markers).

Using this approach, SI protection by rat and mouse brain RNAs results in three bands representing probe fragments whose lengths, based on the sequence of cDNA clones for APP 695, 751, and 770 RNAs are predicted to be 93, 261, and 370 nucleotides, respectively. Although human brain RNAs generate a nearly identical pattern of bands, the band in the APP 751 region appears to migrate more slowly than its rodent counterpart (Figure 1D). Human RNA from the promyelocytic leukemia cell line, HL60, provides a pattern in which APP 695 is barely detectable, APP 770 is present, and APP 751 consists of a doublet (Figure 1D, lane 4). the low ever band of this doublet comigrates with rodent brain APP 751 RNAs (Figure 1D, lanes 6 and 7) and with a labeled fragment protected using an APP 751 clone previously isolated from an HL60 cDNA library (Donnelly, et al., cited above). The upper band of the doublet comigrates with the slowly migrating fragment protected by human brain RNA (Figure 2, lane 5). The upper band, representing the novel RNA of this invention, is observed in 22 of 22 preparations of human brain RNA from multiple individuals.

Example 2

Quantitation of SI Autoradiograms

Autoradiograms of replicate RNA samples subjected to the SI analysis of Example 1 are quantitatively analyzed by densitometry using a Model 300A Computing Densitometer (Molecular Dyanmics, Sunnyvale, CA). Bands representing APP 770, 751 plus 563, 695 and novel RNA are integrated following background subtraction. Ratios of each APP RNA are based on the amount of APP 770 RNA in each sample being set to 1.0. Values represent a ± standard deviation for two determinations.

Using this procedure, the following relative abundance of APP RNAs expressed in cultured human promyelocytic leukemia (HL60) cells and tissue from human, mouse and rat brain are obtained:

| | 695/770 | NOVEL/770 | 770/770 | 751/770 |
|---|---|---|---|---|
| Human Brain | | | | |
| (poly-[$A^+$]) | 16.31 ± 4.76 | 4.37 ± 0.44 | 1.0 | 0.14 ±0.01 |
| (Temporal) | 75.74 ± 28.06 | 27.71 ± 10.54 | 1.0 | 0.21 ±0.05 |
| (Occipital) | 49.24 ± 5.05 | 27.69 ± 6.93 | 1.0 | 0.03 ±0.01 |
| (Parietal) | 101.99 ± 53.35 | 27.61 ± 10.79 | 1.0 | 0.06 ±0.02 |
| Mouse Brain | | | | |
| (poly-[$A^+$]) | 180.34 ± 12.23 | 0.07 ± 0.06 | 1.0 | 5.50 ±1.94 |
| Rat Brain | | | | |
| (poly-[$A^+$]) | 95.26 ± 10.46 | 0.06 ± 0.01 | 1.0 | 7.94 ±2.62 |
| HL60 Cells | | | | |
| (poly-[$A^+$]) | <0.01 | 0.495 ± 0.09 | 1.0 | 0.72 ±0.14 |

Example 3

Further Characterization

To further characterize the novel RNA whose SI protection 15 yields the upper band of the APP 751 doublet described in Example 1, and HL60 cDNA library is rescreened and clones hybriding to a Kunitz protease inhibitor specific oligonucleotide are isolated (using standard techniques as described by Davis, L.G., Dibner, M.D., and Battery, J.F., Basic Methods in Molecular Biology, Elsevier, New York, 1986, pp. 67-78 and summarized in Donnelly, et al., cited above at page 334). Lambda DNA from candidate clones, including the original APP 751 and APP 770 cDNA isolates, are subjected to SI protection analysis as outlined in Example 1.

The results can be seen in Figure 2 (bottom). Labeled riboprobe fragments of three different lengths are protected. Most candidates are of the APP 751 and 770 types, but three clones protected fragments which migrate at the novel band's position. Representative lambda cDNA clone isolates display bands whose fragment lenths are predicted for APP 770 (lanes 6 and 7), APP 751 (lanes 3 and 5), and the novel RNA (lane 4). APP 770 (lane 7) and 751 (lane 5) cDNA isolate have been previously subcloned and sequenced (ibid.). Riboprobe without SI treatment (1 and 2) and markers (lane 1) are also shown.

Each novel lambda clone is found to contain a 1.6 kilobasepair Eco RI fragment. Subcloned, the fragments are found to be identical by sequence analysis accomplished using alkaline denaturation and multiple oligonucleotide primers (described in Ausubel, et al., supra).

Example 4

Protein Expression

A cDNA clone encoding the amyloid peptide precursor of this invention was deposited in the American Type Culture Collection (A.T.C.C.), 12301 Parklawn Drive, Rockville, Maryland 20852, on 22 January 1990, pursuant to the terms of the Budapest Treaty, as a plasmid within HB 101 bacteria. This plasmid, herein referred to as pCLL 365, bears the A.T.C.C. accession number 68207. The DNA and clone are available to the public in the U.S. upon the reqisite issuance of the present application as a U.S. patent and in accordance with other legally approved circumstances.

pCLL is used to express the novel amyloid protein of this invention. To do so, the cDNA insert of pCLL 365 is removed with the appropriate restriction enzymes and placed into the plasmid pET-3a (Rosenberg, A.H., et al., 56 Gene 125 (1987)). The Nde I site of pET-3a is used for the aminoterminal end of the protein and its BAM HI site, for the carboxy-terminal end of the protein. Following transformation of the pET-3a chimera containg APP 365 cDNA into BL-21, LysS bacteria (Studier, F.W., and Moffatt, B.A., 189 J. Mol. Biol. 113 (1986)) protein expression is induced by the addition of lactose or isopropylthiogalactopyranoside. Whole bacteria are then lysed, and APP protein is purified througth conventional protein purification methods.

The foregoing detailed description and examples are further explained and illustrated by the Figures. Thus, Figure 1 schematically illustrates the SI nuclease protection (Example 1) analysis of APP transcripts in human promyelocytic leukemia (HL60) cells and human, mouse and rat brain. (A) depicts construction of the 410-base $^{32}P$-labeled riboprobe depicted in the SI protection strategies in (B) and (C), and used for SI analysis in Figure 2. pAPP770-DA represents a portion of a plasmid constructed by insertion of the 366-base DdeI-XhoI fragment (derived from an APP770 cDNA clone) into the Sma I-XhoI sites of riboprobe vector pIBI30. Transcription of Eco RI-linearized template with $T_3$ RNA polymerase generates a 410-base antisense riboprobe which includes sequences complementary to the Kunitz protease inhibitor (filled box), C (vertical hatched box) and flanking (open box) domains of APP-related RNAs, as well as additional vector sequences (stippled boxes). The riboprobe contains a total of 370 bases complementary to the APP770 transcript; lengths of the relevant sequences are given.

Figure 1 (B) illustrates the predicted SI protection schemes for APP RNAs using theriboprobe described in Figure 1 (A). APP RNAs hybridized to radiolabeled riboprobe are expected to generate SI-resistant probe fragments having lenths of 370, 261, and 93 bases, indicating the presence of APP 770, 751 and 695 RNAs, respectively.

Figure 1 (C) represents the deduced SI protection scheme for the novel RNA of this invention, using the riboprobe described in (A). Based on an aberrant SI pattern in Figure 2 and from the cDNA clone, the novel RNA that is detected is expected to generate at 273-base SI-resistant probe fragment.

Figure 2 (top) shows an autoradiograph of the SI nuclease protection analysis using APP riboprobe in (A) and poly-(A+) or total RNA. Samples include total RNA. Samples include total RNA from human brain temporal cortex (lanes 2 and 3); human promyelocytic leukemia (HL60) cells poly-(A+) (lane 4); and human, mouse, and rat brain poly-(A+) RNAs in lanes 5-7, respectively. Bands corresponding to APP 770, APP 365, and APP 695 are shown in lanes 2-3. In lanes 4-7, the APP 751 region is expanded to better visualize relevant bands corresponding to the SI-protected fragments of variant APP365 and APP751 RNAs. Markers (lane 1) are $^{32}$ p-labeled HaeIII restriction fragments from phiXI74.

Figure 2 (bottom) also compares mobilities of APP365, APP751 and APP770 cDNAs subjected to SI

nuclease protection analysis (described in Example 3). An HL60 cDNA library is screened with a Kunitz protease inhibitor specific probe, and lambda DNA of positive isolates is subjected to SI nuclease protection analysis. Representative lambda cDNA clone isolates display bands having fragment lengths predicted for APP 770 (lanes 6 and 7), APP 751 (lanes 3 and 5) and novel APP 365 (lane 4). Markers (lane 1) are as described above, and riboprobe without SI treatment is also shown (lane 2).

Figure 3 (A) schematically shows the nucleotide and predicted amino acid sequence of a novel APP 365 cDNA of this invention (to be more fully described infra). The organization is oriented in the 5' to 3' direction. The Kunitz protease inhibitor, denoted PI, is found at positions 866 to 1033, and the C domain, at positions 1034 to 1090). The 12-nucleotide deletion (positions 806 to 817) and the novel carboxy-terminus (positions 1091 to 1760) are indicated. Notation below the line represents the number of amino acid residues derived from each domain.

Figure 3 (B) shows the first 670 nucleotides of the insert depicted in Figure 3 (A), with the novel hexameric amino acid sequence at the carboxy-terminus.

Figure 3 (C) shows the nucleotide and predicted amino acid sequende of a novel APP 365 cDNA of this invention. The organization is 5' to 3' starting at position 115 (using the numbering system described by Kange, et al. 325 Nature 733 (1987)) and continuing for 1630 additional nucleotides. The 12-nucleotide deletion is shown with a vertical arrow between nucleotides 805 and 806. Double underlined residues represent the Kunitz protease inhibitor region, and single underlined residues represent the novel hexameric carboxyterminus.

The above description is for the purpose of teaching the person of ordinary skill in the art how to practice the present invention, and it is not intended to detail all those obvious modifications and variations of it which will become apparent to the skilled worker upon reading the description. It is intended, however, that all such obvious modifications and variations be included within the scope of the present invention as defined in appended claims.

## BIBLIOGRAPHY


Aufdembrinke, B., et al., U.S. Pat. No. 4,711,891 (1987).
Ausubel, F.M., et al. eds., Current Protocols in Molecular Biology, John Wiley & Sons, New York, 1989, 3.12.1 to 3.12.3.
Bendotti, C., et al., 85 Proc. Nat. Acad. Sci. U.S.A. 3628 (1988).
Card, J.P., et al., 1 Neuron 835 (1988).
Chirgwin, et al. 24 Biochem. 5294 (1979).
Cole, G., et al., 100 Neurosci. Lett. 340 (1989).
Davis, L.G., Dibner, M.D., and Battey, J.F., Basic Methods in Molecular Biology, Elsevier, New York, 1986, pages 67-78.
DeSavage, F., and Octave, J.N., 245 Science 651 (1989).
Devereaux, J., et al., 12 Nucleic Acids Res. 387 (1986).
Donnelly, R.J., et al., 9 Neurobiol. Agining 333 (1988).
Dyrks, T., et al., 7 EMBO 949 (1988).
Furuya, H., et al., 150 Biochem. Biophys. Res. Commun. 75 (1988).
Glenner, G.G., and Wong, C., 112 Biochem. Biophys. Res. Commun. 1131 (1984).
Gordon, P., U.S. Pat. No. 3,646,007 (1972)
Hardy, J.A., et al., 7 Neurobiol. Aging 489 (1986).
Kang, J., et al., 325 Nature 733 (1987).
Kang, J. and Muller-Hill, B., 17 Nucleic Acid Res 2130 (1989).
Katzman, R., 33 Arch. Neurol. 217 (1976).
Kitaguchi, N., et al, 331 Nature 530 (1988).
Laskowski, M., and Kato, I., 49 Ann Rev. Biochem. 593 (1980).
Lemaire, H.G., et al., 17 Nucleic Acid Res. 517 (1989).
Lindberg, U.H.A., and Oegren S.O., U.S. Pat. No.4,469,707 (1984).
Masters, C., et al., 82 Proc. Nat. Acad. Sci. U.S.A. 4245 (1985).
Monard, D., Trends Neurosci. 11: 541-544, 1988
Murdoch, G., et al. 16 Nucleic Acids Res. 357 (1988).
Podlisny, M., et al. 238 Science 669 (1987).
Ponte, P., et al., 331 Nature 525 (1988).
Roch, M., et al., 209 Nature 109 (1966).
Rosenberg, A.H., et al., 56 Gene 125 (1987).

Sambrook, J., E.F., Fritsch, and T. Maniatis, Molecular Cloning: A Laboratroy Manual, 2nd ed., Cold Spring Harbor Laboratory Press, 1989, 10.29 to 10.37.
Scangos, G., et al., Eur. Pat. Ap. No. 341,491 (1989).
St. George-Hyslop, P.H., et al., 235 Science 235 (1987).
Studier, F.W., and Moffatt, B.A., 189 J. Mol. Biol. 113 (1986).
Selkoe, D.J., et al., 85 Proc. Nat. Acad. Sci. U.S.A. 7341 (1988).
Tanzi, R., et al., 331 Nature 528 (1988).
Tuttle, R.R., U.S. Pat. No. 4,511,570 (1985).
Vitek, M.P., et al., 4 Mol. Brain Res. 121 (1988).
Wong, C., et al., 82 Proc. Nat. Acad. Sci. U.S.A. 8729 (1982).
Wyngaarden, J.B., and Smith, L.H., eds., Cecil's Textbook of Medicine, 18th ed., W.B. Saunders, 1989, pages 2089 to 2090.
Zain, S.B., et al. 85 Proc. Nat Acad. Sci. U.S.A. 929 (1988).
Zurn, A. D. et al., Dev. Neurosci. 10: 17-24, 1988.

## Claims

**1.** A purified and isolated RNA sequence coding for a soluble human brain tissue amyloid peptide precursor protein.

**2.** A purified and isolated RNA sequence selected from the group consisting of:
(a) RNA sequences that will hybridize to the DNA sequence set out in Figure 3C; and
(b) RNA sequences that code for soluble brain tissue amyloid protein precursors sufficiently duplicative of the protein encoded by the DNA of Figure 3C to allow possession of the biological properties of said protein.
(c) an RNA sequence encoded by the DNA sequence set out in Figure 3C.

**3.** A purified and isolated DNA sequence coding for a soluble human brain tissue amyloid peptide precursor protein.

**4.** A purfied and isolated DNA sequence selected from the groups consisiting of:
(a) DNA sequences that will hybridize to the DNA sequence set out in Figure 3C; and
(b) DNA sequences that code for soluble brain tissue amyloid protein precursors sufficiently duplicative of the protein encoded by the DNA of Figure 3C to allow possession of the biological properties of said protein; and
(c) a DNA sequence corresponding to the DNA sequence set out in Figure 3C.

**5.** A biologically functional circular plasmid or viral DNA vector comprising a DNA sequence according to claim for 3 or 4.

**6.** A procaryotic or eucaryotic host cell transformed or transfected with a DNA sequence according to claim 14 in a manner allowing the host cell to express a human amyloid precursor protein.

**7.** A biologically functional plasmid pCLL 365, A.T.C.C., Rockville, Maryland, Accession No. 68207.

**8.** A bacterial host cell transformed with the DNA encoded in the plasmid according to claim 7 in a manner allowing said host cell to express a human amyloid precursor protein.

**9.** A purified and isolated soluble human brain tissue amyloid peptide precursor protein.

**10.** A purified and isolated soluble brain tissue amyloid protein precursor sufficiently duplicative of the soluble brain tissue amyloid protein precursor encoded by the DNA of Figure 3C to allow possession of the biological properties of said protein, or comprising the amino acid sequence encoded by the DNA of Figure 3C.

**11.** A method for producing soluble brain tissue amyloid protein precursor, comprising propagating host cells transformed or transfected with a purified and isolated DNA sequence coding for a soluble human brain tissue amyloid peptide precursor protein in culture medium for a time and under conditions sufficient for said host cells to express said protein precursor, and therefrom recovering soluble brain tissue amyloid protein precursor from materials selected from the group consisting of said culture medium, lysates of said host cells, and membrane fractions of said host cells.

**12.** A method for producing procaryotic or eucaryotic cells expressing a soluble brain tissue amyloid protein precursor, comprising transfecting or transforming procaryotic or eucaryotic host cells with a DNA sequence encoding a soluble human brain tissue amyloid peptide precursor protein in a manner allowing said host cell to express the protein encoded by said DNA.

**13.** A method for stimulating nerve cell growth in vivo or in vitro comprising applying to the nerve cells an effective amount of APP-365 protein, or active portion thereof.

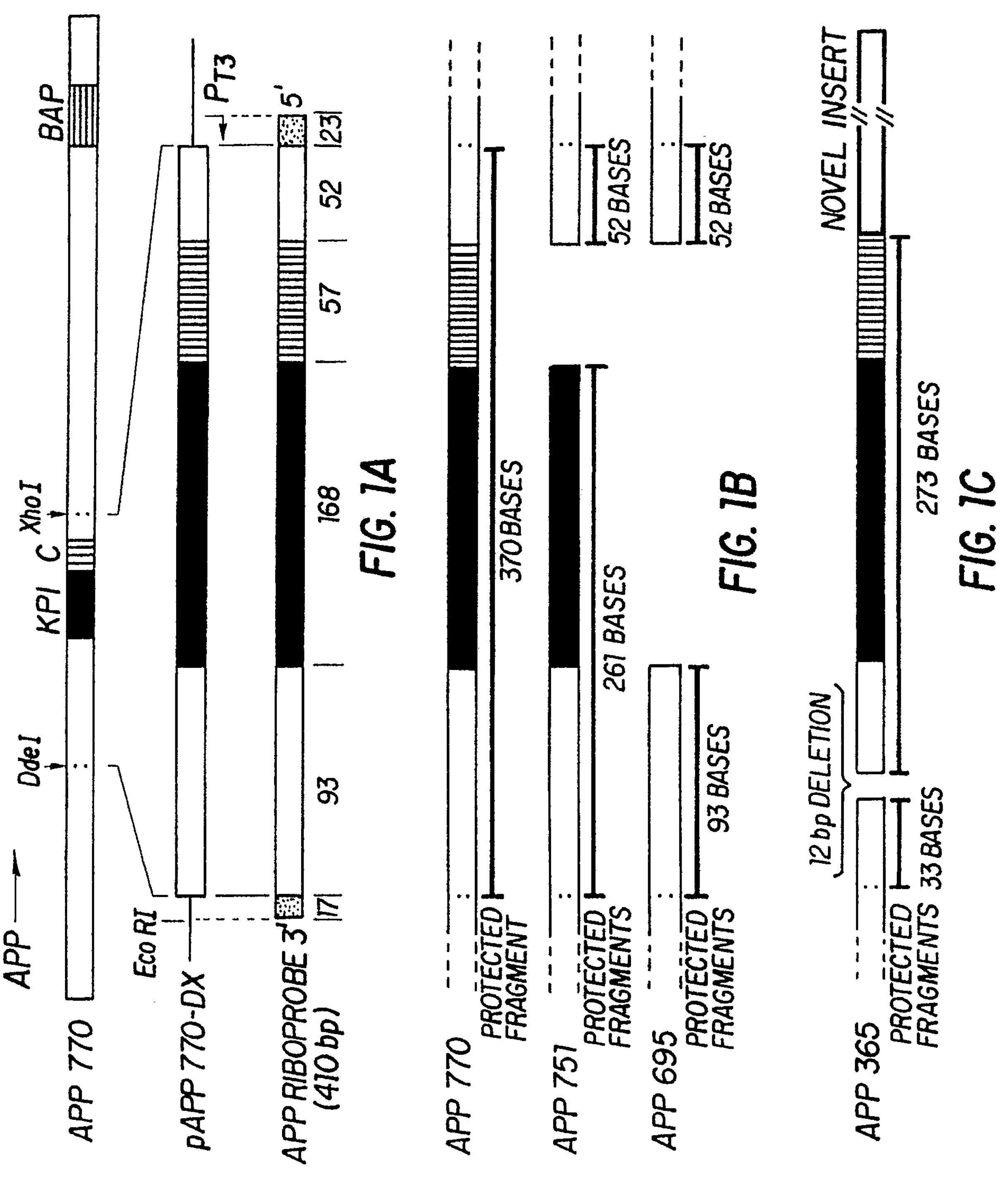


FIG. 1A

FIG. 1B

FIG. 1C

# FIG. 2

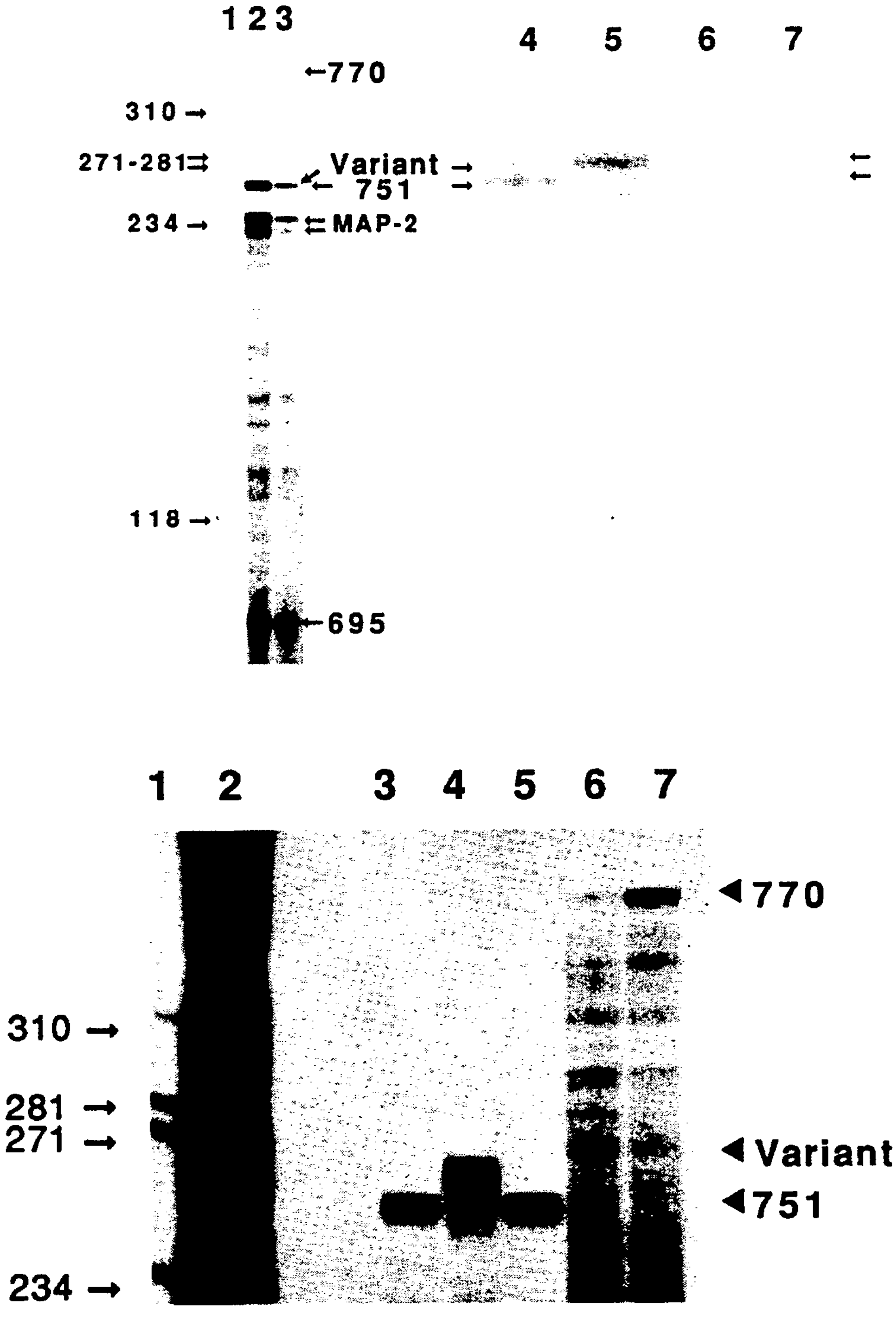
1 2 3
4 5 6 7
←770
310 →
271-281⇉
Variant →
751 →
234 →
⇇MAP-2
118 →
←695
1 2 3 4 5 6 7
310 →
281 →
271 →
234 →
◀770
◀Variant
◀751

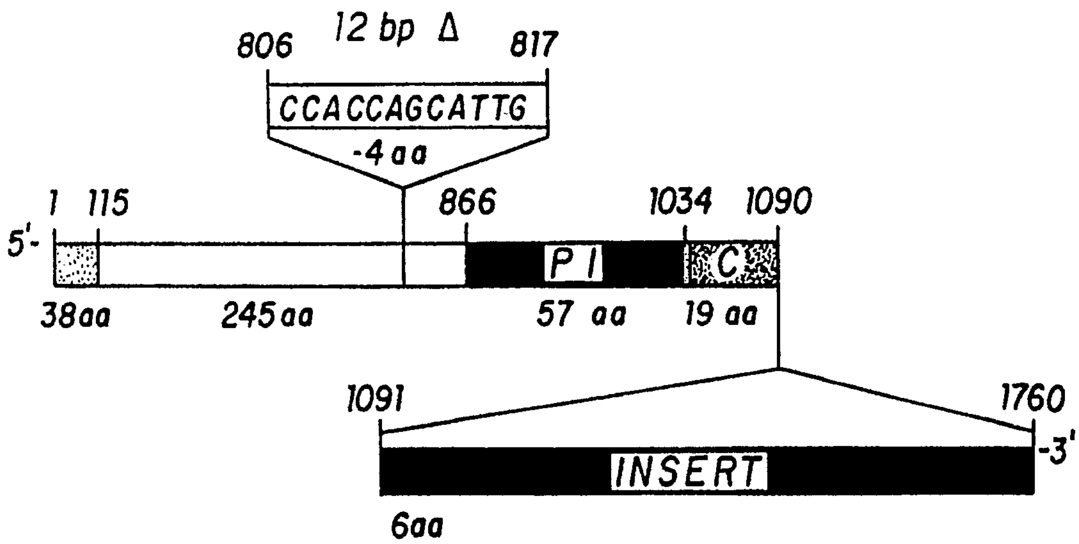


FIG. 3A

```
1091  CGTACGTTGT CATTCACCTG AGGGAAGGGA AGAGGGGAGG AGGATGCTGC
      ArgThrLeuS erPheThrEN D
1141  TTGGTTCACA TAACTCCAGC ATCATCACCT TCTTTGCATG GTTTTGTGTT
1191  TCTTGAACAC CTGTCTTAGT AAAATGTTTC TTCCCATTAC CTTGCTTGTA
1241  ATTACATCTG ATTTTGCCAG ACAGCTTGAG ATGTTGGGCT AAGAACATCA
1291  TTGACTAAGT TTCTTCTATT TCTGACCAAT TTCCTTTTTA TTTAGTCTGG
1341  TTTTATTGAA TATTATGTGG ACAACATCAT TGTATTGTAT TTGCCATTAC
1391  TATTTTATTT CCTAAAAGCT CTCAGTGTAA CTGAGAGCAG GCTTAGCCTC
1441  TCACTGCTTT TGCAGAACTG AAGAACAAGG GCTAGGTGCA GTGGAAGGAA
1491  AGTGACTTTA CTTAGCAAAG CTAGCAATGG GGAAATGGTC CAGGCTCCTG
1541  CCTTAAAGCA ACCATCTCAA ATTTTGGATT AAAAACAAAG GCTTAAAAAG
1591  GGGAGCTTGG AATGCAGGGC ATGAAGGAGG GGTGAGGAGG TGCTGCTATA
1641  CAGGACTTGT TCCAAAGACT TGAGTTATTA TCCAGTTCGG TAAGTGGGCT
1691  GGCGCATCCC GCACAATCGG GTTGTAAATT AACTGCAGCC TTGAGGTGAT
1741  CTCCTGATGA GGGAGAATTC
```

FIG. 3B

```
                 120                 140                 160
    GAATTCGG GGCAGACTGAACATGCACATGAATGTCCAGAATGGGAAGTGGGATTCAGATC
             GlyArgLeuAsnMetHisMetAsnValGlnAsnGlyLysTrpAspSerAspP
             40

             180                 200                 220
167 CATCAGGGACCAAAACCTGCATTGATACCAAGGAAGGCATCCTGCAGTATTGCCAAGAAG
    roSerGlyThrLysThrCysIleAspThrLysGluGlyIleLeuGlnTyrCysGlnGluV
             60

             240                 260                 280
227 TCTACCCTGAACTGCAGATCACCAATGTGGTAGAAGCCAACCAACCAGTGACCATCCAGA
    alTyrProGluLeuGlnIleThrAsnValValGluAlaAsnGlnProValThrIleGlnA
             80

             300                 320                 340
287 ACTGGTGCAAGCGGGGCCGCAAGCAGTGCAAGACCCATCCCCACTTTGTGATTCCCTACC
    snTrpCysLysArgGlyArgLysGlnCysLysThrHisProHisPheValIleProTyrA
             100

             360                 380                 400
347 GCTGCTTAGTTGGTGAGTTTGTAAGTGATGCCCTTCTCGTTCCTGACAAGTGCAAATTCT
    rgCysLeuValGlyGluPheValSerAspAlaLeuLeuValProAspLysCysLysPheL
             120

             420                 440                 460
407 TACACCAGGAGAGGATGGATGTTTGCGAAACTCATCTTCACTGGCACACCGTCGCCAAAG
    euHisGlnGluArgMetAspValCysGluThrHisLeuHisTrpHisThrValAlaLysG
             140

             480                 500                 520
467 AGACATGCAGTGAGAAGAGTACCAACTTGCATGACTACGGCATGTTGCTGCCCTGCGGAA
    luThrCysSerGluLysSerThrAsnLeuHisAspTyrGlyMetLeuLeuProCysGlyI
             160

             540                 560                 580
527 TTGACAAGTTCCGAGGGGTAGAGTTTGTGTGTTGCCCACTGGCTGAAGAAAGTGACAATG
    leAspLysPheArgGlyValGluPheValCyscysProLeuAlaGluGluSerAspAsnV
             180

             600                 620                 640
587 TGGATTCTGCTGATGCGGAGGAGGATGACTCGGATGTCTGGTGGGGCGGAGCAGACACAG
    alAspSerAlaAspAlaGluGluAspAspSerAspValTrpTrpGlyGlyAlaAspThrA
             200

             660                 680                 700
647 ACTATGCAGATGGGAGTGAAGACAAAGTAGTAGAAGTAGCAGAGGAGGAAGAAGTGGCTG
    spTyrAlaAspGlySerGluAspLysValValGluValAlaGluGluGluValAlaG
             220

             720                 740                 760
707 AGGTGGAAGAAGAAGAAGCCGATGATGACGAGGACGATGAGGATGGTGATGAGGTAGAGG
    luValGluGluGluGluAlaAspAspAspGluAspAspGluAspGlyAspGluValGluG
             240

             780                 800                 820
767 AAGAGGCTGAGGAACCCTACGAAGAAGCCACAGAGAGAACCACCACCACCACCACCACCA
    luGluAlaGluGluProTyrGluGluAlaThrGluArgThrThrThrThrThrThrThrT
             260
```

FIG. 3C

```
                840                 860                 880
827     CAGAGTCTGTGGAAGAGGTGGTTCGAGAGGTGTGCTCTGAACAAGCCGAGACGGGGCCGT
        hrGluSerValGluGluValValArgGluValCysSerGluGlnAlaGluThrGlyProC
                280

                900                 920                 940
887     GCCGAGCAATGATCTCCCGCTGGTACTTTGATGTGACTGAAGGGAAGTGTGCCCCATTCT
        ysArgAlaMetIleSerArgTrpTyrPheAspValThrGluGlyLysCysAlaProPheP
                300

                960                 980                 1000
947     TTTACGGCGGATGTGGCGGCAACCGGAACAACTTTGACACAGAAGAGTACTGCATGGCCG
        heTyrGlyGlyCysGlyGlyAsnArgAsnAsnPheAspThrGluGluTyrCysMetAlaV
                320

                1020                1040                1060
1007    TGTGTGGCAGCGCCATGTCCCAAAGTTTACTCAAGACTACCCAGGAACCTCTTGCCCGAG
        alCysGlySerAlaMetSerGlnSerLeuLeuLysThrThrGlnGluProLeuAlaArgA
                340

                1080                1100                1120
1067    ATCCTGTTAAACGTACGTTGTCATTCACCTGA GGGAAGGGAAGAGGGGAGGAGGATGCTG
        spProValLysArgThrLeuSerPheThrEnd
                360

         1140                  1160                1180
CTTGGTTCACATAACTCCAGCATCATCACCTTCTTTGCATGGTTTTGTGTTTCTTGAACACCTGTCT

   1200                1220                1240                1260
TAGTAAAATGTTTCTTCCCATTACCTTGCTTGTAATTACATCTGATTTTGCCAGACAGCTTGAGATG

                1280                1300                1320
TTGGGCTAAGAACATCATTGACTAAGTTTCTTCTATTTCTGACCAATTTCCTTTTTATTTAGTCTGG

        1340                1360                1380
TTTTATTGAATATTATGTGGACAACATCATTGTATTGTATTTGCCATTACTATTTTATTTCCTAAAA

   1400                1420                1440                1460
GCTCTCAGTGTAACTGAGAGCAGGCTTAGCCTCTCACTGCTTTTGCAGAACTGAAGAACAAGGGCTA

               1480                1500                1520
GGTGCAGTGGAAGGAAAGTGACTTTACTTAGCAAAGCTAGCAATGGGGAAATGGTCCAGGCTCCTGC

        1540                1560                1580
CTTAAAGCAACCATCTCAAATTTTGGATTAAAAACAAAGGCTTAAAAGGGAGCTTGGAATGCAGGGC

  1600                1620                1640                1660
ATGAAGGAGGGGTGAGGAGGTGCTGCTATACAGGACTTGTTCCAAAGACTTGAGTTATTATCCAGTT

             1680                1700                1720
CGGTAAGTGGGCTGGCGCATCCCGCACAATCGGTTGTAAATTAACTGCAGCCTTGAGGTGATCTCC

       1740
TGATGAGGGAGAATTC
```

*FIG. 3C Cont.*